# EUROPEAN PATENT SPECIFICATION

(11) **EP 2 408 396 B1**
(45) Date of publication and mention of the grant of the patent: **21.03.2018**
(21) Application number: 10754240.9
(22) Date of filing: 22.03.2010
(51) Int. Cl.: A61C 1/08, A61N 5/06, A61C 19/06, A61C 17/02

(54) **ORAL IRRIGATOR APPLIANCE WITH RADIANT ENERGY DELIVERY FOR BACTERICIDAL EFFECT**
MUNDDUSCHE MIT STRAHLUNGSENERGIEVERABREICHUNG FÜR BAKTERIENABTÖTENDE WIRKUNG
APPAREIL IRRIGATEUR BUCCAL AVEC DÉLIVRANCE D'ÉNERGIE RAYONNANTE POUR EFFET BACTÉRICIDE

(30) Priority: 20.03.2009 US 162126 P
(43) Date of publication of application: 25.01.2012
(73) Proprietor: Water Pik, Inc., Fort Collins, CO 80553-0001 (US)
(72) Inventor: SNYDER, Clifford, J., Fort Collins, CO 80525 (US); HASZIER, Gordon, Fort Collins, CO 80521 (US); LUETTGEN, Harold, Windsor, CO 80550 (US); HAIR, Kenneth A., Fort Collins, CO 80526 (US); COVER, Daniel Bernard, Fort Collins, CO 80528 (US)
(74) Representative: Miller, James Lionel Woolverton
(86) International application number: PCT/US2010/028180
(87) International publication number: WO 2010/108189

(56) References cited:
- US-A- 5 795 153
- US-A- 5 795 153
- US-A1- 2005 064 371
- US-A1- 2008 008 979
- US-A1- 2008 008 979
- US-A1- 2009 070 949
- US-A1- 2009 070 949
- US-B2- 6 561 808
- US-B2- 6 561 808

## Description

### TECHNICAL FIELD

This technology relates to an oral irrigator, and more particularly to an oral irrigator including a radiant energy source to enhance the bacteria reducing effect.

### BACKGROUND

An oral irrigator, also referred to as a dental water jet, includes generally a water reservoir supplying water to a pump, which in turn delivers water through a handle member having a tip structure, and into a user's mouth. The tip structure is sized and oriented to allow the user to direct the water stream against the user's teeth or gums as desired. The water stream may be continuous or pulsed. The reservoir of the oral irrigator may be positioned on a counter top, or may be hand held. Examples of such oral irrigators are described in U.S. Patent nos. 6,056,710 and 7,147,468 and U.S. Patent Application Publication No. 2008/0008979.

US2005/0064371 describes a device capable of exposing the oral cavity of a subject to a therapeutic amount of light, to improve oral health.

The effectiveness of existing oral irrigators is derived by the disruptive influence of the water stream on the bacteria found in the mouth. The bacteria is dislodged by the water stream and delivered out of the mouth (either swallowed or rinsed out). [0005] The information included in this Background section of the specification, including any references cited herein and any description or discussion thereof, is included for technical reference purposes only and is not to be regarded subject matter by which the scope of the invention is to be bound.

### SUMMARY

The invention relates to an oral irrigator as defined in the claims. In one implementation, an oral irrigator for delivery radiant energy includes a base housing, a pump mechanism, a reservoir operably associated with the base housing and fluidically associated with the pump mechanism, a jet tip fluidically associated with the reservoir that directs a fluid at a surface inside an oral cavity; and a radiant energy source directing radiant energy at a surface inside an oral cavity. According to the invention, the radiant energy source and the jet tip are unitary to direct both the fluid and the radiant energy in generally the same direction.

The oral irrigator for delivering radiant energy includes a radiant energy conduit that directs the radiant energy from the radiant energy source to the oral cavity. The radiant energy conduit and the fluid conduit are unitary and form the jet tip to direct both the fluid and the radiant energy from the same terminal point in generally the same direction.

In one implementation, the oral irrigator may be a handheld device with the jet tip, the radiant energy source, and the reservoir in one body for easy maneuverability or use when traveling.

This Summary is provided to introduce a selection of concepts in a simplified form that are further described below in the Detailed Description. Other features, details, utilities, and advantages of the present invention will be apparent from the following more particular written description of various embodiments of the invention as further illustrated in the accompanying drawings and defined in the appended claims.

### BRIEF DESCRIPTION OF THE DRAWINGS

Fig. 1A is an isometric view of an implementation of an oral irrigator of the disclosure including a jet tip emitting radiant energy.
Fig. 1B is an enlarged view of a terminal end of the jet tip of the oral irrigator shown in Fig. 1A.
Fig. 2A is an isometric view of an alternate implementation of an oral irrigator of the disclosure including a jet tip for emitting radiant energy.
Fig. 2B is an enlarged view of the terminal end of the jet tip of the oral irrigator shown in Fig. 2A.
Fig. 3A is an isometric view of a jet tip of a further implementation of an oral irrigator of the disclosure, wherein the radiant energy source is in the handle and radiant energy is transmitted via a light tube to the terminal end of the jet tip.
Fig. 3B is an enlarged view of a handheld oral irrigator of the disclosure, wherein the radiant energy source is in the handle and radiant energy is transmitted via a light tube to the terminal end of the jet tip.
Figs. 4A is an isometric view of an implementation of an oral irrigator of the disclosure for emitting radiant energy including a jet handle and tip for fluid discharge and a separate handle for radiant energy application.
Fig. 4B is an isometric view depicting the oral irrigator of Fig. 4A with the jet handle and tip removed from the base housing and reservoir unit.
Figs. 5A is an isometric view of an implementation of an oral irrigator of the disclosure for emitting radiant energy with a single jet handle and tip includes both a fluid conduit for directing fluid and an additional radiant energy conduit for directing radiant energy from collocated terminal ends.
Fig. 5B is an isometric view depicting the oral irrigator of Fig. 5A with the jet handle and tip removed from the base housing and reservoir unit and the radiant energy conduit of the oral irrigator activated.
Fig. 5C is an enlarged partial view of the collocated radiant energy conduit tip and jet tip of Fig. 5A.
Fig. 6A is a schematic diagram of a collocated fluid conduit and radiant energy conduit for an oral irrigator jet tip of the disclosure.
Fig. 6B is an isometric view of a molded lens system for focusing light energy into the radiant energy conduit of Fig. 6A.
Fig. 7 is a bar graph depicting the effects of an implementation of an oral irrigator with a radiant energy delivery system on undesirable black pigmented bacteria as opposed to desirable non-black pigmented bacteria in a typical oral cavity.
Fig. 8A is a side elevation view of an implementation of an oral irrigator jet tip of the disclosure that forms an integral radiant energy conduit.
Fig. 8B is a front elevation view of the oral irrigator jet tip of Fig. 8A.
Fig. 8C is a bottom plan view of the oral irrigator jet tip of Fig. 8A.
Fig. 8D is a cross section of the oral irrigator jet tip of Fig. 8B taken along lines A-A.
Fig. 9A is a graph depicting the incoherent irradiance measured at a detector imparted by an oral irrigator tip of the implementation of Figs. 8A-8D in which the jet tip is formed as an integral radiant energy conduit and the radiant energy is transmitted without a corresponding water stream.
Fig. 9B is a detector image of the incoherent irradiance levels graphed in Fig. 9A.
Fig. 10A is a graph depicting the incoherent irradiance measured at a detector imparted by an oral irrigator tip of the implementation of Figs. 8A-8D in which the jet tip is formed as an integral radiant energy conduit and the radiant energy is transmitted in conjunction with a corresponding water stream.
Fig. 10B is a detector image of the incoherent irradiance levels graphed in Fig. 10A.
Fig. 11A is a graph depicting the incoherent illuminance measured at a detector imparted by an oral irrigator tip of the implementation of Figs. 8A-8D in which the jet tip is formed of a tube of PMMA and the radiant energy is transmitted without a corresponding water stream.
Fig. 11B is a detector image of the incoherent illuminance levels graphed in Fig. 11A.
Fig. 12 is a side elevation view of an oral irrigator jet handle according to the claimed invention with a radiant energy source transmitted via a light guide positioned coaxially within a fluid conduit of the jet tip.
Fig. 13 is a cross-section view of the oral irrigator jet handle of Fig. 12 taken along line 13-13.
Fig. 14 is an isometric view of a light guide used in the jet handle of the oral irrigator of Fig. 12.
Fig. 15 is a cross-section view of the light guide of Fig. 14 taken along line 15-15.
Fig. 16 is an isometric view of a collimator used in the jet handle of the oral irrigator of Fig. 12.
Fig. 17 is a bottom plan view of the collimator of Fig. 16.
Fig. 18 is a side elevation view of the collimator of Fig. 16.
Fig. 19 is a cross-section view of the collimator of Fig. 16 taken along line 19-19 of Fig. 18.

### DETAILED DESCRIPTION

The technology disclosed herein pertains generally to the enhancement of the effectiveness of the traditional oral irrigator. In particular, the impact of the water stream from the jet tip is enhanced by the addition of a radiant energy source that also works to reduce the bacteria in a user's mouth without also using chemical additives. The wavelength of radiant energy is selected to closely match the adsorption peaks of certain black-pigmented oral bacteria. The radiant energy source may be located in any number of positions so long as it is directed at least partially into the user's oral cavity when the oral irrigator is used.

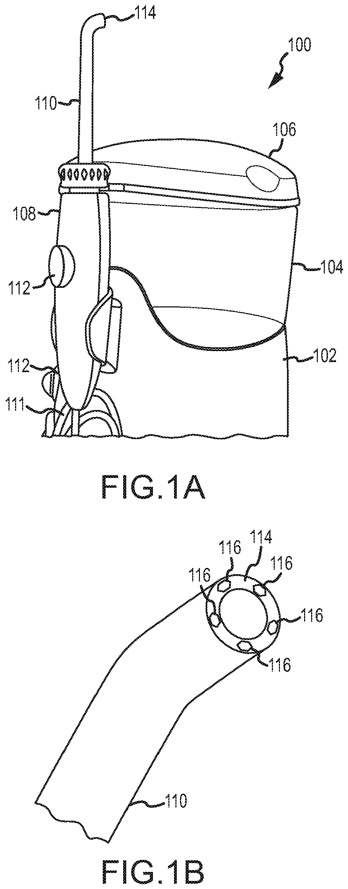

Figs. 1A and 1B depict an implementation of an oral irrigator with a radiant energy delivery system 100. An oral irrigator 100 is shown having a base housing 102, which incorporates the pump powered by line voltage. A reservoir 104 having a lid sits atop the base housing 102 and serves to supply the water to the jet tip 110. The reservoir 104 is fluidically connected to the pump in order to pump water through a water line 111 to the jet handle 108. The jet tip 110 is fluidically connected to the jet handle 108 so that the pumped water flows through the jet tip 110. The jet tip 110 has a terminal end 114 that is positioned so as to cause the water stream to enter the oral cavity and flush bacteria therefrom.

The radiant energy, in this instance in the form of a light emitting diode (LED) emitting light in the 350 to 450 nanometer range, preferably in the 375-415 nm range, and even more preferably in the 405-415 nm range, is configured relative to the terminal end 114 of the jet tip 110 so the radiant energy is generally directed in at least a similar direction as the water stream.

As shown in the example of Fig. 1B, the radiant energy is created by five surface-mount LEDs 116 positioned around the terminal end 114 of the jet tip. Each of the surface-mount LEDs 116 are electrically connected to a power source, typically the same as the one that powers the pump in the base housing 102. In one example, the electrical connections are wires extending from each LED 116 to a common wire, which then extends down the jet tip 110, along the handle 108, along the water line 111 to the base housing 102. In another example, the common wire may be embedded in a sidewall of the jet tip 110 and further in a sidewall of the water line 111. In other examples, the LEDs 116 may be connected in series.

Controls 112 may be positioned on the handle 108 and/or base housing 102 to control the pressure and other characteristics of the water stream, as well as characteristics of the LEDs 112, for example, activation, deactivation, intensity level, and activation time, among other options.

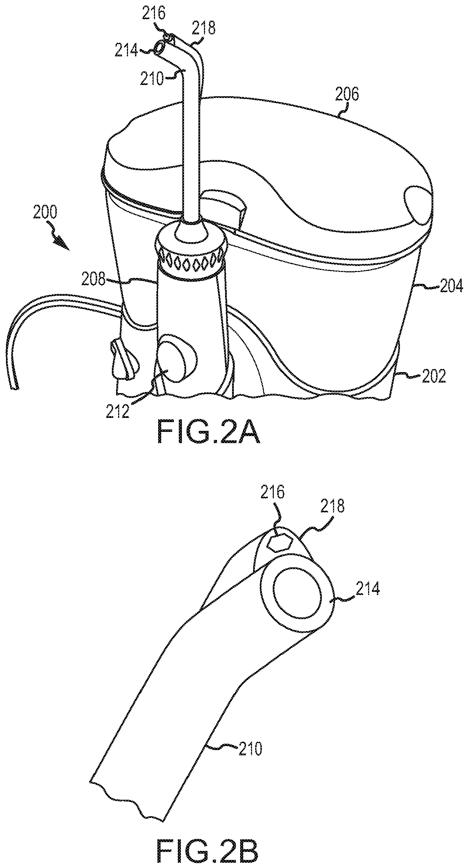

Figs. 2A and 2B depict an alternative implementation of an oral irrigator 200 with a radiant energy delivery system. As in the prior figures, the oral irrigator 200 is composed of a base housing 202, a fluid reservoir 204, a lid 206, a handle 208, a jet tip 210, and one or more controls or actuators 212. In this implementation a single LED 216 is attached to one side of the terminal end 214 of the jet tip 210. The LED 216 is mounted on a shoulder 218 formed on the terminal end 214 of the jet tip 210. This design makes the terminal end 214 of the jet tip 210 a slightly larger in one dimension compared to a standard jet tip. The LED 216 is energized by lead wires contained or enveloped within the wall of jet tip 210. In other embodiments, the LED 216 may be a surface mount configuration that connects with a receptacle formed in the shoulder 218 or otherwise on the terminal end 214 of the jet tip 210.

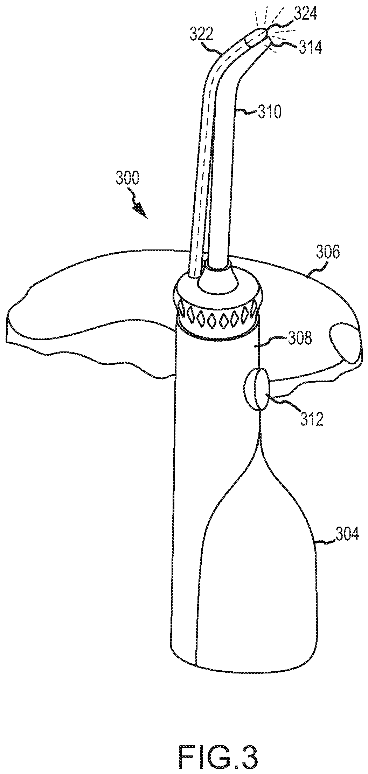

In an alternative implementation as shown in Figs. 3A and 3B, the radiant light source may be positioned remote from the terminal end 314 of the jet tip 310 and directed along the jet tip 310 for use. For example, as shown in Fig. 3A, the radiant light source of the oral irrigator 300 is positioned on the handle 308 with the radiant energy transmitted to the terminal end 314 of the jet tip 310 by a radiant energy conduit 322, e.g., a light tube. The energy 322 may be terminated at a location 324 at or adjacent the terminal end 314 of the jet tip 310. Alternatively, the termination location 324 of the radiant energy conduit 322 at a length shorter or longer than the terminal end 314 of the jet tip 310. In the example of Fig. 3B, the oral irrigator 300' is a handheld configuration with the reservoir 304' mounted to the handle 308'. The radiant energy source may be mounted in the handle 308' and powered by the portable power supply (e.g., a rechargeable battery) contained within the handle 308'. In this example, the handle 308' acts as a base, and includes a water pump mechanism and a control switch. The power source powers the pump mechanism and the radiant energy source. The control switch controls the power to the pump mechanism and/or the radiant energy source to actuate or deactivate the respective function. These functions may also be controlled by separate control switches.

In various implementations, the radiant energy conduit 322 may be a light tube made of glass or plastic and may also include or be formed of optical fibers. In one example, the light tube may be formed of poly(methyl methacrylate) (PMMA). In another example, the light tube may be formed as a glass or plastic fiber-optic light injector. The examples of Figs. 3A and 3B allow the light source to be positioned remote from the terminal end 314 of the jet tip 310 to allow an LED or non-LED energy source to be used and to reduce exposure of the light source to moisture and physical impact with the user's oral cavity or other objects.

The radiant energy conduit 322 may also be aimed to cast the radiant energy in the same direction as the jet tip 310 to converge at the same location as the water stream exiting the jet tip 310, or the radiant energy may be directed generally in the same direction or in a different direction if desired. The radiant energy conduit 322 may also be selectively positionable to allow the user to adjust the position. The radiant energy may be directed or focused to shine in the same area of impact of the water jet in order to take advantage of the water jet lifting away the gum from the tooth and allowing the radiant energy to reach bacteria below the gum line.

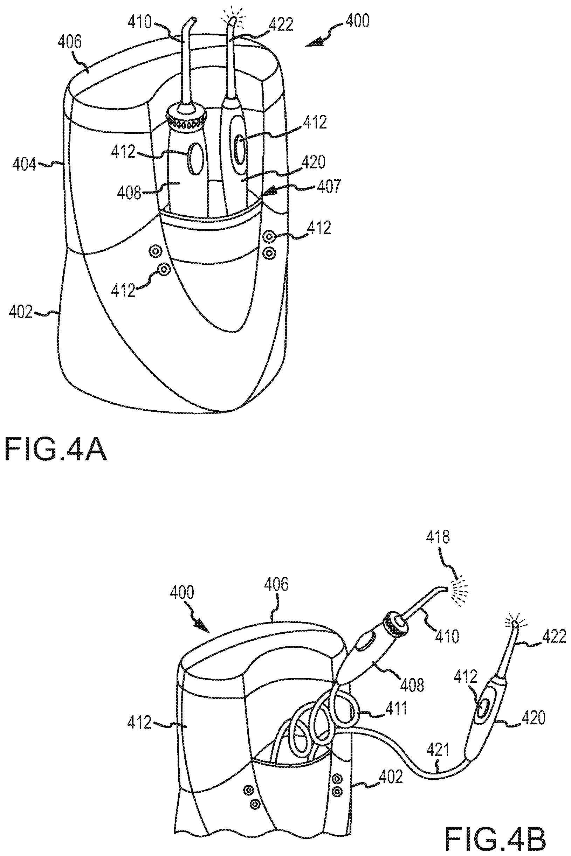

Figs. 4A-4D depict another implementation of an oral irrigator 400 in which a water jet handle 408 operates to provide a water stream 418, while a separate delivery wand 420 operates to provide the application of radiant light through a radiant energy conduit 422. The base 402 of the oral irrigator 400 supports a reservoir 404 covered by a lid 406 and a storage recess 407 for holding the handle 408 and the wand 420. The water jet handle 408 includes a jet tip 410 and a water line 411 communicating fluid from the pump to the jet tip 410 (as described above). Controls 412 on the base 402 and the water jet handle 408 allow some control of the characteristics of the water stream.

Still referring to Figs. 4A-D, the radiant energy delivery wand 420 is provided for directing the radiant energy through the radiant energy conduit 422 into the user's oral cavity. The separate energy delivery wand 420 is connected to a power source at the base 402 by a power cord 421. In an alternate example, the energy delivery wand 420 may be battery powered and not require a cord 421. The energy delivery wand 420 may include a switch 412 for controlling the status of the radiant energy, for example, activation and deactivation, and may also function to set the intensity level of the radiant energy.

The water jet handle 408 may be removed from the storage recess 407 in the base 402 and extended for use by the user to direct the water stream 418 into the user's mouth as depicted in Fig. 4C. The energy delivery wand 420 may similarly be removed from the storage recess 407 in the base 402 and extended for use by the user to direct the radiant energy through the radiant energy conduit 422 into the user's mouth as shown in Fig. 4D.

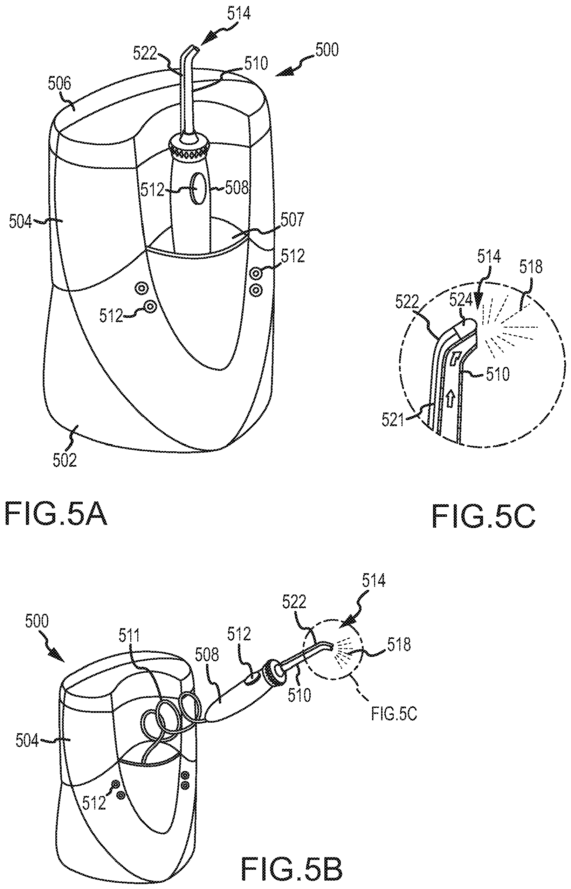

Figs. 5A-5E depict another implementation of an oral irrigator 500. The oral irrigator 500 includes a base 502 for supporting a reservoir 504 having a lid 506 and a single jet handle 508. The jet handle 508 includes a jet tip 510 formed as a fluid conduit for directing a flow of water out of a terminal end 514 of the jet tip 510. The jet handle 508 also includes radiant energy source 524 positioned near the terminal end 514 of the jet tip 510. The radiant energy source 524 is positioned to direct light in at least generally the same direction of the terminal end 514 of the jet tip 510. In this example, the radiant energy source 524 is positioned at the end of a second conduit 522 running along the length of the water conduit 510. An electrical wire 521 runs along the second conduit 522, in this case within the interior cavity of the second conduit 522, to provide power to the radiant energy source 524 positioned at the tip of the second conduit 522 as best shown in Fig. 5E.

As shown in Figs. 5-5D, the jet handle 508 includes a switch 512 to control the water flow through the first water conduit 510. The same switch 512 may also control the activation, deactivation, and intensity condition of the radiant energy source 524. Alternately, each may be controlled by a switch 512 positioned elsewhere on the unit, for example, on the base 502. The use of this oral irrigator device 500 may allow a user separate use of the water jet tip 510 and radiant energy source 524, or may allow the simultaneous use thereof.

In each of the above examples (as well as further examples below), the radiant energy sources may be suitably constructed to activate when the water flow is actuated, or may be controlled by sensors to actuate when positioned in a relatively dark space (such as the inside of a user's mouth), or may be controlled by a timer to help insure sufficient radiant energy is imparted to the bacteria in the user's mouth.

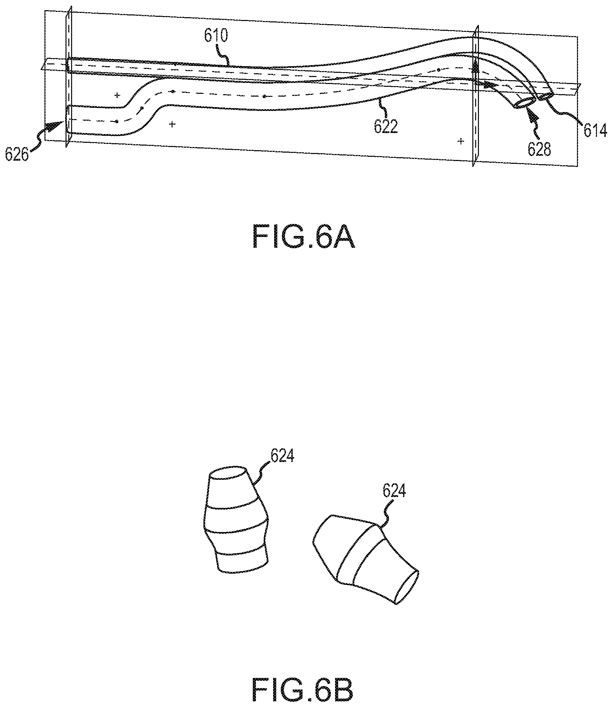

Fig. 6A schematically depicts an alternate example of a jet tip with a water conduit 610 separate from a corresponding radiant energy conduit 622. The water conduit 610 and the energy conduit 622 generally follow parallel paths and are mounted adjacent each other. The terminal end 614 of the water conduit 610 is at approximately the same distance from the handle as the distal end 628 of the energy conduit 622. In this example, the energy conduit is a glass or plastic shaft or cylinder, or possibly a fiber optic light injector that transmits radiant energy from a light source at a proximal end 626 of the energy conduit 622 to the distal end 628 of the light conduit 622. Fig. 6B is a photograph depicting two commonly available molded acrylic fiber light injectors 624 from Fraen Corporation.

Exemplary LEDs may include, for example, Nichia 5POA (375 nm), Nichia 59013 (365 nm), or Xicon 351-3314-RC LEDs. In some implementations, suitable wavelengths for effective radiant energy have been found between 350-450 nm, preferably between 375-415nm, even more preferably between 405-415 nm. In one exemplary implementation, a UV-1WS-L2 LED from Prolight Opto Technology Corporation was used to provide light at desired wavelengths. Another way to characterize effective radiant energy is by intensity. The effective intensity required will depend on the species of microbe. Minimum effective intensities generally range from 2-50 J/cm.

The following tables present test results from the use of various LEDs and other light sources for varying amounts of time on various common types of bacteria that inhabit the oral cavity to determine the bactericidal effects. The Legend indicates the types of bacteria used in the experiments, the types of LEDs used, and an explanation of the meaning of the results. In the first experiment of Table 1, bacteria cultures were exposed to the light sources for periods of 2 minutes and 60 minutes. In the experiments of Tables 2, 3, and 4, bacteria cultures were exposed to the light sources for periods of 5 seconds, 30 seconds, 1 minute, 2 minutes, and 60 minutes. As indicated in the Legend, an IE or "Ineffective" entry means bacterial growth was observed in the culture without apparent inhibition, i.e., the incident light did not kill the bacteria. In contrast, an E or "Effective" entry indicates that while live bacteria remain in the culture, the bacteria were killed in the illuminated area.

| **Legend for Tables 1-4** | |
|---|---|
| NG = No growth on plate - invalid data point | |
| IE = "Ineffective" - Bacterial growth on plate but no inhibition zones | |
| E = "Effective" - Bacteria growth on plate but bacteria killed in area illuminated | |
| | |
| Bacteria 1 Porphyromonas Gingivalis ATCC 33277 | |
| Bacteria 2 Prevotella Intermedia ATCC 25611 | |
| Bacteria 3 Prevotella Nigrescens ATCC 33563 | |
| Bacteria 4 Prevotella Melaningena ATCC 25845 | |
| | |
| led 1 | Nichia 59013 - 365 nm |
| led 2 | Mouser UV Xicon Led Lamps Taiwan PN-351-3314-RC |
| led 3 | Blue - Sunbright 470 nm-ssp-lx6144A7uc |
| led 4 | Nichia - 5poa-375 nm |
| led 5 | White - Sunbright-ssp-lx6144A9UC |
| led 6 | UV Florescent-JKL |
| led 7 | FOX-uv |
| led 8 | IR vcsel |

| **Table 1 - Bacteria 1** | | | |
|---|---|---|---|
| Light Source | | 2 min | 60 min |
| Control | | IE | IE (poor) |
| Black Light | | IE | |
| Germicidal | | E | E |
| filter 1 | | IE ? | |
| filter 2 | | IE ? | |
| led 1 | | IE ? | E |
| led 2 | | IE ? | E |
| led 3 | | IE ? | ? |
| led 4 | | IE ? | ? |
| led 5 | | IE | |
| led 6 | | IE | |
| led 7 | | IE | |
| led 8 | | IE | |

| **Table 2 - Bacteria 2** | | | | | |
|---|---|---|---|---|---|
| Light Source | 5 sec | 30 sec | 60 sec | 2 min | 60 min |
| Control | IE | IE | IE | IE | IE |
| Black Light | | | | IE | E |
| Germicidal | E | E | E | E | E |
| filter 1 | | | | IE | E |
| filter 2 | | | | IE | E |
| led 1 | IE | E (partial) | IE | E | E |
| led 2 | IE | IE | IE | E | E |
| led 3 | IE | IE | IE | IE | E |
| led 4 | E | E | E | E | E |
| led 5 | | | | IE | E |
| led 6 | | | | IE | IE |
| led 7 | | | | IE | E |
| led 8 | | | | | |

| **Table 3 - Bacteria 3** | | | | | |
|---|---|---|---|---|---|
| Light Source | 5 sec | 30 sec | 60 sec | 2 min | 60 min |
| Control | IE | IE | IE | IE | IE |
| Black Light | | | | IE | E |
| Germicidal | E | E | E | E | E |
| filter 1 | | | | IE | E |
| filter 2 | | | | IE | E |
| led 1 | IE | IE | E | E | E |
| led 2 | IE | IE | IE | E | E |
| led 3 | IE | IE | IE | IE | E |
| led 4 | IE | E | E | E | E |
| led 5 | | | | IE | E |
| led 6 | | | | IE | IE |
| led 7 | | | | IE | E |
| led 8 | | | | IE | IE |

| **Table 4 - Bacteria 4** | | | | | |
|---|---|---|---|---|---|
| Light Source | 5 sec | 30 sec | 60 sec | 2 min | 60 min |
| Control | IE | IE | IE | IE | IE |
| Black Light | | | | IE | IE |
| Germicidal | E | E | E | E | E |
| filter 1 | | | | IE | IE |
| filter 2 | | | | IE | IE |
| led 1 | IE | IE | E (partial) | E | E |
| led 2 | IE | IE | IE | IE | E |
| led 3 | IE | IE | IE | E ? | E ? |
| led 4 | IE | E | E | E ? | E ? |
| led 5 | | | | IE | E |
| led 6 | | | | IE | IE |
| led 7 | | | | IE | IE |
| led 8 | | | | IE | IE |

In addition to the experimental testing above, another series of tests of radiant energy sources was performed to determine the effects of alternate energy sources. In the experiments of Tables 5, 6, 7, and 8, bacteria cultures were exposed to the light sources for periods of 5 seconds, 30 seconds, 1 minute, 2 minutes, and 60 minutes. As in the prior experiments, an IE or "Ineffective" entry means bacterial growth was observed in the culture without apparent inhibition. In contrast, an E or "Effective" entry indicates that while live bacteria remain in the culture, the bacteria were killed in the illuminated area.

| **Table 5** - **Light Effects on Porphyromonas Gingivalis** | | | | | | | |
|---|---|---|---|---|---|---|---|
| Source (nm) | Configuration | Plate | Light # | 5 sec | 30 sec | 2 min | 5 min |
| 405 | 30E leaded | A | 1 | IE | IE | IE | IE |
| 420 | 15E leaded | A | 2 | IE | IE | IE | IE |
| | (5) Nichia 590 a | A | 3 | IE | IE | IE | IE |
| | (4) 0603 surface mount | A | 4 | IE | IE | IE | IE |
| 395 | L300 CUV Ledtronics | B | 1 | IE | IE | IE | IE |
| 395 | L120 CUV Ledtronics | B | 2 | IE | IE | IE | IE |
| 405 | SPL300CUV | B | 3 | IE | IE | IE | IE |
| 405 | L200CUV | B | 4 | IE | IE | IE | IE |
| 375 | Nichia into 2mm fiber | C | 1 | IE | IE | IE | IE |
| | broken into 1 mm | C | 2 | IE | IE | IE | IE |
| | module 1 mm | C | 3 | IE | IE | IE | IE |
| 420 | 15E leaded | C | 4 | IE | IE | IE | IE |
| 375 | Nichia into 1 mm fiber | C | 5 | IE | IE | IE | IE |
| 408 | 18E into 1mm | C | 6 | IE | IE | IE | IE |
| 375 | nichia into 1 mm | C | 7 | IE | IE | IE | IE |
| 394 | filtered sunlight | S | 1 | IE | IE | IE | IE |
| 400 | filtered sunlight | S | 2 | IE | IE | IE | IE |
| 405 | filtered sunlight | S | 3 | IE | IE | IE | IE |
| 410 | filtered sunlight | S | 4 | IE | IE | IE | IE |
| 415 | filtered sunlight | S | 5 | IE | IE | IE | IE |
| 254 | Sterilizing wand | W | 1 | IE | E | E | E |

| **Table 6** - **Light Effects on Prevotella Intermedia** | | | | | | | |
|---|---|---|---|---|---|---|---|
| Source (nm) | Configuration | Plate | Light # | 5 sec | 30 sec | 2 min | 5 min |
| 405 | 30E leaded | A | 1 | IE | E | IE | E |
| 420 | 15E leaded | A | 2 | IE | IE | IE | IE |
| | (5) Nichia 590 a | A | 3 | IE | IE | E | E |
| | (4) 0603 surface mount | A | 4 | IE | IE | IE | IE |
| 395 | L300 CUV Ledtronics | B | 1 | IE | E | E | E |
| 395 | L120CUV Ledtronics | B | 2 | IE | E | E | E |
| 405 | SPL300CUV | B | 3 | IE | E | E | E |
| 405 | L200CUV | B | 4 | IE | E | E | E |
| 375 | Nichia into 2mm fiber | C | 1 | IE | IE | IE | IE |
| | broken into 1 mm | C | 2 | IE | IE | IE | IE |
| | module 1 mm | C | 3 | IE | E | E | E |
| 420 | 15E leaded | C | 4 | IE | IE | IE | IE |
| 375 | Nichia into 1 mm fiber | C | 5 | IE | IE | IE | IE |
| 408 | 18E into 1mm | C | 6 | IE | IE | IE | IE |
| 375 | nichia into 1mm | C | 7 | IE | IE | IE | IE |
| 394 | filtered sunlight | S | 1 | IE | IE | IE | IE |
| 400 | filtered sunlight | S | 2 | IE | IE | IE | IE |
| 405 | filtered sunlight | S | 3 | IE | IE | IE | IE |
| 410 | filtered sunlight | S | 4 | IE | IE | IE | IE |
| 415 | filtered sunlight | S | 5 | IE | IE | IE | IE |
| 254 | Sterilizing wand | W | 1 | E | E | E | E |

| **Table 7 - Light Effects on Prevotella Nigrescens** | | | | | | | |
|---|---|---|---|---|---|---|---|
| Source (nm) | Configuration | Plate | Light # | 5 sec | 30 sec | 2 min | 5 min |
| 405 | 30E leaded | A | 1 | E | E | E | E |
| 420 | 15E leaded | A | 2 | IE | IE | IE | IE |
| | (5) Nichia 590 a | A | 3 | E | E | E | E |
| | (4) 0603 surface mount | A | 4 | IE | IE | E | E |
| 395 | L300 CUV Ledtronics | B | 1 | E | E | E | E |
| 395 | L120 CUV Ledtronics | B | 2 | E | E | E | E |
| 405 | SPL300CUV | B | 3 | E | E | E | E |
| 405 | L200CUV | B | 4 | E | E | E | E |
| 375 | Nichia into 2mm fiber | C | 1 | IE | IE | E | E |
| | broken into 1 mm | C | 2 | IE | IE | IE | IE |
| | module 1 mm | C | 3 | E | E | E | E |
| 420 | 15E leaded | C | 4 | E | E | E | E |
| 375 | Nichia into 1 mm fiber | C | 5 | IE | IE | E | E |
| 408 | 18E into 1mm | C | 6 | IE | IE | IE | IE |
| 375 | nichia into 1 mm | C | 7 | IE | IE | E | E |
| 394 | filtered sunlight | S | 1 | IE | IE | E | E |
| 400 | filtered sunlight | S | 2 | IE | IE | E | E |
| 405 | filtered sunlight | S | 3 | IE | IE | IE | E |
| 410 | filtered sunlight | S | 4 | IE | IE | IE | E |
| 415 | filtered sunlight | S | 5 | IE | IE | IE | E |
| 254 | Sterilizing wand | W | 1 | E | E | E | E |

| **Table 8 - Light Effects on Prevotella Melaningena** | | | | | | | |
|---|---|---|---|---|---|---|---|
| Source | Configuration | Plate | Light | 5 sec | 30 | 2 min | 5 min |
| (nm) | | | # | | sec | | |
| 405 | 30E leaded | A | 1 | IE | IE | E | E |
| 420 | 15E leaded | A | 2 | IE | IE | IE | IE |
| | (5) Nichia 590 a | A | 3 | IE | E | E | E |
| | (4) 0603 surface mount | A | 4 | IE | IE | IE | IE |
| 395 | L300 CUV Ledtronics | B | 1 | IE | IE | E | E |
| 395 | L120 CUV Ledtronics | B | 2 | IE | E | E | E |
| 405 | SPL300CUV | B | 3 | IE | IE | E | E |
| 405 | L200CUV | B | 4 | E | E | E | E |
| 375 | Nichia into 2mm fiber | C | 1 | IE | IE | IE | IE |
| | broken into 1 mm | C | 2 | IE | IE | IE | IE |
| | module 1 mm | C | 3 | IE | IE | ES | E |
| 420 | 15E leaded | C | 4 | IE | IE | IE | IE |
| 375 | Nichia into 1 mm fiber | C | 5 | IE | IE | IE | IE |
| 408 | 18E into 1mm | C | 6 | IE | IE | IE | IE |
| 375 | nichia into 1 mm | C | 7 | IE | IE | IE | IE |
| 394 | filtered sunlight | S | 1 | IE | IE | IE | IE |
| 400 | filtered sunlight | S | 2 | IE | IE | IE | IE |
| 405 | filtered sunlight | S | 3 | IE | IE | IE | IE |
| 410 | filtered sunlight | S | 4 | IE | IE | IE | IE |
| 415 | filtered sunlight | S | 5 | IE | IE | IE | IE |
| 254 | Sterilizing wand | W | 1 | IE | E | E | E |

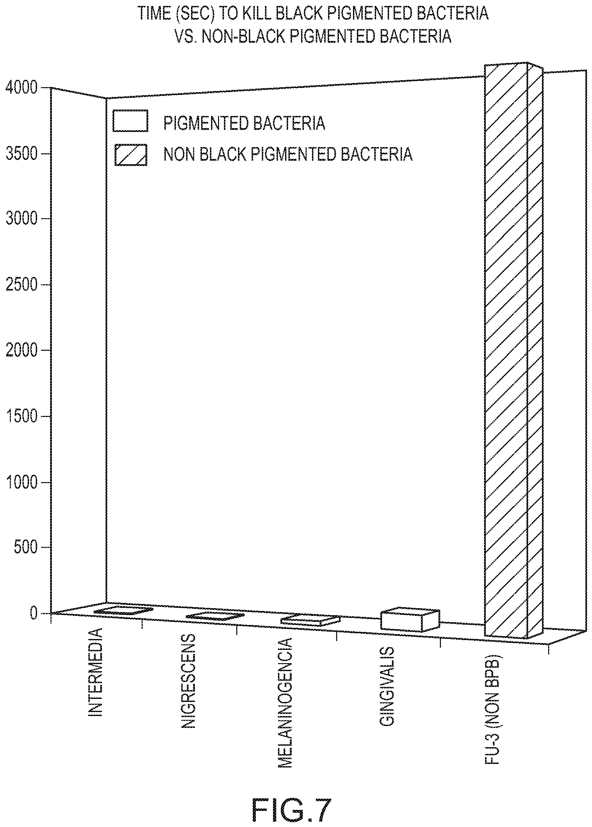

These studies indicate that UV and near-UV light is effective in killing select periodontal pathogens. While shorter wavelength UV radiation is an extremely effective germicide, the mechanism of destruction in UV radiation below 300 nm is to destroy DNA in cells. (See, e.g., Soukos, N.S. et al., Phototargeting oral black-pigmented bateria, Antimicrobial Agents and Chemotherapy, (April 2005) pp. 1391-96.) This mechanism is not selective and therefore the user's tissue cells could be destroyed as well. In contrast, by using higher wavelengths of light, e.g., between 350-450 nanometers, undesirable, black-pigmented bacteria can be destroyed without affecting the health of adjacent oral tissue. Wavelengths between 350-450 nm, and especially between 405-415 nm, are very effective bactericides by exciting endogenous porphyrins within the black-pigmented bacteria while leaving oral tissue unharmed. Fig. 7 is a bar graph showing the effectiveness of a 405 nm light source on black-pigmented bacteria compared to non-black-pigmented bacteria, which is actually healthy to have in the oral cavity. The undesirable black-pigmented bacteria are killed relatively quickly (in some cases under 5 seconds) while the desirable bacteria remains unharmed. This selective killing when used on a daily basis causes a beneficial, long-term shift in the ratio of desirable to undesirable bacteria as the desirable bacteria are allowed to grow and take the place previously occupied by the undesirable bacteria. This results in a lasting benefit to the user's oral health beyond what would be indicated by the one-time kill efficacy.

In examples using a light tube 622 as a radiant energy conduit as in Fig. 6A to direct the radiant energy from an energy source 624, the light tube 622 may be formed from plastic or glass fibers with a transmissive core and optionally a thin sheathing a material that has a lower refractive index, e.g., Mitsubishi Eska acrylic fibers sheathed with fluorine polymer, or similar glass fibers. Molded light tubes from acrylic polymers are common in many manufactured products. One example is the glowing speedometer needle of most modern automobiles. Fiber optic light injectors could also be used as light tubes. In another implementation, a molded light injector, e.g., as commercially produced by Fraen Corporation, may be used to direct light from an LED into an optical fiber or molded light tube.

Additional tests were performed to gauge the efficacy of various light sources on a number of common oral bacteria and other organisms commonly found in the oral cavity. Results of these tests are set forth below in Tables 9A-16B and are summarized in Table 17. In each table pair, the first table designated "A" shows the results of various exposures using a fiber optic radiant energy source. In the second tables of the pairs designated "B", results of various exposures using a radiant energy source mounted at the tip of the device are presented. In the tables, a "+" indicates no inhibition of the organism to the light source, a "W" indicates a weak inhibition of the organism to the light source, and a "-" indicates an inhibition of the organism to the light source.

Tables 9A-9B depict the results of exposure of Porphyromonas gingivalis ATCC 33277 (PG-1) to various light sources for periods of time between 5 seconds and 45 minutes (900 seconds). PG-1 is an anaerobic black pigmented bacteria associated with periodontal disease. In Table 9A, results of exposure to no light, and fiber optic sources of white light, FI Pro Light-2mm, and AWP Pro Light-2mm are depicted. PG-1 is one of the most resistant organisms, but testing shows first kills in some experiments within between 60 and 120 seconds of exposure. In Table 9B, results of exposure to tip mounted light sources at dominant wavelengths of 400 nm (two samples), 590 nm, and a surface mount white light are presented.

| Table 9A - PG-1 with Fiber Optic Source | | | | | | |
|---|---|---|---|---|---|---|
| Organism | Plate | Time | White light-3mm | No Light | FI Pro Light-2mm | AWP Pro Light-2mm |
| PG-1 | A | 5 Sec | - | - | - | - |
| PG-1 | A | 15 Sec | - | - | - | - |
| PG-1 | A | 30 Sec | - | - | - | - |
| PG-1 | A | 60 Sec | - | - | - | - |
| PG-1 | A | 2 Min | **+** | - | - | **W** |
| PG-1 | A | 15 min | **+** | - | **+** | no data |
| PG-1 | A | 45 Min | **+** | - | **+** | no data |

| Table 9B - PG-1 with Tip Mounted Source | | | | | | |
|---|---|---|---|---|---|---|
| Organism | Plate | Time | 400 nm | 400 nm | 590 A | Surface mount |
| PG-1 | B | 5 Sec | - | - | - | - |
| PG-1 | B | 15 Sec | - | - | - | - |
| PG-1 | B | 30 Sec | - | - | - | - |
| PG-1 | B | 60 Sec | - | - | - | - |
| PG-1 | B | 2 Min | - | - | - | - |
| PG-1 | B | 15 min | **+** | - | - | **+** |
| PG-1 | B | 45 Min | **+** | **+** | **+** | **+** |

Tables 10A-10B depict the results of exposure of Prevotella melaninogenica ATCC 258465 (PM-2) to various light sources for periods of time between 5 seconds and 45 minutes (900 seconds). PM-2 is an anaerobic black pigmented bacteria associated with periodontal disease. In Table 10A, results of exposure to no light, and fiber optic sources of white light, FI Pro Light-2mm, and AWP Pro Light-2mm are depicted. In Table 10B, results of exposure to tip mounted light sources at dominant wavelengths of 400 nm (two samples), 590 nm, and a surface mount white light are presented.

| Table 10A - PM-2 with Fiber Optic Source | | | | | | |
|---|---|---|---|---|---|---|
| Organism | Plate | Time | White light-3mm | No Light | FI Pro Light-2mm | AWP Pro Light-2mm |
| PM-2 | A | 5 Sec | - | - | - | - |
| PM-2 | A | 15 Sec | - | - | - | - |
| PM-2 | A | 30 Sec | - | - | **+** | **W** |
| PM-2 | A | 60 Sec | - | - | **+** | **+** |
| PM-2 | A | 2 Min | **+** | - | **+** | **+** |
| PM-2 | A | 15 min | **+** | - | **+** | no data |
| PM-2 | A | 45 Min | **+** | - | **+** | no data |

| Table 10B - PM-2 with Tip Mounted Source | | | | | | |
|---|---|---|---|---|---|---|
| Organism | Plate | Time | 400 nm | 400 nm | 590 A | Surface mount |
| PM-2 | B | 5 Sec | - | - | - | - |
| PM-2 | B | 15 Sec | **W** | - | - | - |
| PM-2 | B | 30 Sec | **+** | - | - | **W** |
| PM-2 | B | 60 Sec | **+** | **W** | - | **+** |
| PM-2 | B | 2 Min | **+** | **+** | - | **+** |
| PM-2 | B | 15 min | **+** | **+** | **+** | **+** |
| PM-2 | B | 45 Min | **+** | **+** | **+** | **+** |

Tables 11A-11B depict the results of exposure of Porphyromonas Intermedia ATCC 25611 (PI-1) to various light sources for periods of time between 5 seconds and 45 minutes (900 seconds). PI-1 is an anaerobic black pigmented bacteria associated with periodontal disease. Comments in literature and the experimentation conducted herein suggests that PI-1 tends to be more susceptible to UV and less susceptible to antibiotics than P. Ginvivalis. In Table 11A, results of exposure to no light, and fiber optic sources of white light, FI Pro Light-2mm, and AWP Pro Light-2mm are depicted. In Table 11B, results of exposure to tip mounted light sources at dominant wavelengths of 400 nm (two samples), 590 nm, and a surface mount white light are presented.

| Table 11A - PI-1 with Fiber Optic Source | | | | | | |
|---|---|---|---|---|---|---|
| Organism | Plate | Time | White light-3mm | No Light | FI Pro Light-2mm | AWP Pro Light-2mm |
| PI-1 | A | 5 Sec | **+** | - | **+** | **+** |
| PI-1 | A | 15 Sec | **+** | - | **+** | **+** |
| PI-1 | A | 30 Sec | **+** | - | **+** | **+** |
| PI-1 | A | 60 Sec | **+** | - | **+** | **+** |
| PI-1 | A | 2 Min | **+** | - | **+** | **+** |
| PI-1 | A | 15 min | **+** | - | **+** | **+** |
| PI-1 | A | 45 Min | **+** | - | **+** | **+** |

| Table 11B - PI-1 with Tip Mounted Source | | | | | | |
|---|---|---|---|---|---|---|
| Organism | Plate | Time | 400 nm | 400 nm | 590 A | Surface mount |
| PI-1 | B | 5 Sec | **+** | **-** | **-** | **+** |
| PI-1 | B | 15 Sec | **+** | **+** | **+** | **+** |
| PI-1 | B | 30 Sec | **+** | **+** | **+** | **+** |
| PI-1 | B | 60 Sec | **+** | **+** | **+** | **+** |
| PI-1 | B | 2 Min | **+** | **+** | **+** | **+** |
| PI-1 | B | 15 min | **+** | **+** | **+** | **+** |
| PI-1 | B | 45 Min | **+** | **+** | **+** | **+** |

Tables 12A-12B depict the results of exposure of Porphyromonas Nigrescens ATCC 33563 (PN-1) to various light sources for periods of time between 5 seconds and 45 minutes (900 seconds). PN-1 is an anaerobic black pigmented bacteria associated with periodontal disease. Comments in literature and the experimentation conducted herein suggests that PN-1 tends to be more susceptible to UV and less susceptible to antibiotics than P. Ginvivalis. In Table 12A, results of exposure to no light, and fiber optic sources of white light, FI Pro Light-2mm, and AWP Pro Light-2mm are depicted. In Table 12B, results of exposure to tip mounted light sources at dominant wavelengths of 400 nm (two samples), 590 nm, and a surface mount white light are presented.

| Table 12A - PN-1 with Fiber Optic Source | | | | | | |
|---|---|---|---|---|---|---|
| Organism | Plate | Time | White light-3mm | No Light | FI Pro Light-2mm | AWP Pro Light-2mm |
| PN-1 | A (BA) | 5 Sec | **+** | - | **+** | **+** |
| PN-1 | A (BA) | 15 Sec | **+** | - | **+** | **+** |
| PN-1 | A (BA) | 30 Sec | **+** | - | **+** | **+** |
| PN-1 | A (BA) | 60 Sec | **+** | - | **+** | **+** |
| PN-1 | A (BA) | 2 Min | **+** | - | **+** | **+** |
| PN-1 | A (BA) | 15 min | **+** | - | **+** | no data |
| PN-1 | A (BA) | 45 Min | **+** | - | **+** | no data |

| Table 12B - PN-1 with Tip Mounted Source | | | | | | |
|---|---|---|---|---|---|---|
| Organism | Plate | Time | 400 nm | 400 nm | 590 nm | Surface mount |
| PN-1 | B (BA) | 5 Sec | **+** | **W** | **-** | **+** |
| PN-1 | B (BA) | 15 Sec | **+** | **+** | **W** | **+** |
| PN-1 | B (BA) | 30 Sec | **+** | **+** | **+** | **+** |
| PN-1 | B (BA) | 60 Sec | **+** | **+** | **+** | **+** |
| PN-1 | B (BA) | 2 Min | **+** | **+** | **+** | **+** |
| PN-1 | B (BA) | 15 min | **+** | **+** | **+** | **+** |
| PN-1 | B (BA) | 45 Min | **+** | **+** | **+** | **+** |

Tables 13A-13B depict the results of exposure of Streptococcus mutans ATCC 25175 (STR-54) to various light sources for periods of time between 5 seconds and 45 minutes (900 seconds). STR-54 is a gram-positive, facultatively anaerobic bacteria commonly found in the human oral cavity. In Table 13A, results of exposure to no light, and fiber optic sources of white light, FI Pro Light-2mm, and AWP Pro Light-2mm are depicted. In Table 13B, results of exposure to tip mounted light sources at dominant wavelengths of 400 nm (two samples), 590 nm, and a surface mount white light are presented.

| Table 13A - STR-54 with Fiber Optic Source | | | | | | |
|---|---|---|---|---|---|---|
| Organism | Plate | Time | White light-3mm | No Light | FI Pro Light-2mm | AWP Pro Light-2mm |
| Str-54 | A (BA) | 5 Sec | - | - | - | - |
| Str-54 | A (BA) | 15 Sec | - | - | - | - |
| Str-54 | A (BA) | 30 Sec | - | - | - | - |
| Str-54 | A (BA) | 60 Sec | - | - | - | - |
| Str-54 | A (BA) | 2 Min | **+** | - | - | **+** |
| Str-54 | A (BA) | 15 min | **+** | - | **W** | no data |
| Str-54 | A (BA) | 45 Min | **+** | - | **+** | no data |

| Table 13B - STR-54 with Tip Mounted Source | | | | | | |
|---|---|---|---|---|---|---|
| Organism | Plate | Time | 400 nm | 400 nm | 590 A | Surface mount |
| Str-54 | B (BA) | 5 Sec | - | - | - | - |
| Str-54 | B (BA) | 15 Sec | **-** | **-** | **-** | **-** |
| Str-54 | B (BA) | 30 Sec | - | **-** | **-** | **-** |
| Str-54 | B (BA) | 60 Sec | - | **-** | **-** | **-** |
| Str-54 | B (BA) | 2 Min | **W** | - | - | - |
| Str-54 | B (BA) | 15 min | **W** | - | **W** | **-** |
| Str-54 | B (BA) | 45 Min | **+** | - | **W** | **W** |

Tables 14A-14B depict the results of exposure of Lactobacillus casei ATCC 393 (LB-2) to various light sources for periods of time between 5 seconds and 45 minutes (900 seconds). LB-2 is a stain agent common in milk and dairy products and is associated with carries formation. In Table 14A, results of exposure to no light, and fiber optic sources of white light, FI Pro Light-2mm, and AWP Pro Light-2mm are depicted. In Table 14B, results of exposure to tip mounted light sources at dominant wavelengths of 400 nm (two samples), 590 nm, and a surface mount white light are presented.

| Table 14A - LB-2 with Fiber Optic Source | | | | | | |
|---|---|---|---|---|---|---|
| Organism | Plate | Time | White light-3mm | No Light | FI Pro Light-2mm | AWP Pro Light-2mm |
| LB-2 | A (BA) | 5 Sec | - | - | - | - |
| LB-2 | A (BA) | 15 Sec | - | - | - | - |
| LB-2 | A (BA) | 30 Sec | - | - | - | - |
| LB-2 | A (BA) | 60 Sec | - | - | - | - |
| LB-2 | A (BA) | 2 Min | - | - | - | - |
| LB-2 | A (BA) | 15 min | - | - | - | - |
| LB-2 | A (BA) | 45 Min | **+** | - | **+** | no data |

| Table 14B - LB-2 with Tip Mounted Source | | | | | | |
|---|---|---|---|---|---|---|
| Organism | Plate | Time | 400 nm | 400 nm | 590 A | Surface mount |
| LB-2 | B (BA) | 5 Sec | - | - | - | - |
| LB-2 | B (BA) | 15 Sec | - | - | - | - |
| LB-2 | B (BA) | 30 Sec | - | - | - | - |
| LB-2 | B (BA) | 60 Sec | - | - | - | - |
| LB-2 | B (BA) | 2 Min | - | - | - | - |
| LB-2 | B (BA) | 15 min | - | - | - | - |
| LB-2 | B (BA) | 45 Min | **+** | - | - | - |

Tables 15A-15B depict the results of exposure of Actinobacillus actinomycetemcomitans ATCC 33384 (AA-1) to various light sources for periods of time between 5 seconds and 45 minutes (900 seconds). AA-1 is a bacteria associated with periodontal disease. In Table 15A, results of exposure to no light, and fiber optic sources of white light, FI Pro Light-2mm, and AWP Pro Light-2mm are depicted. In Table 15B, results of exposure to tip mounted light sources at dominant wavelengths of 400 nm (two samples), 590 nm, and a surface mount white light are presented.

| Table 15A - AA-1 with Fiber Optic Source | | | | | | |
|---|---|---|---|---|---|---|
| Organism | Plate | Time | White light-3mm | No Light | FI Pro Light-2mm | AWP Pro Light-2mm |
| AA-1 | A (BA) | 5 Sec | - | - | - | - |
| AA-1 | A (BA) | 15 Sec | - | - | - | - |
| AA-1 | A (BA) | 30 Sec | - | - | - | - |
| AA-1 | A (BA) | 60 Sec | - | - | - | - |
| AA-1 | A (BA) | 2 Min | - | - | **+** | **+** |
| AA-1 | A (BA) | 15 min | **+** | **-** | **+** | no data |
| AA-1 | A (BA) | 45 Min | **+** | - | **+** | no data |

| Table 15B - AA-1 with Tip Mounted Source | | | | | | |
|---|---|---|---|---|---|---|
| Organism | Plate | Time | 400 nm | 400 nm | 590 A | Surface mount |
| AA-1 | B (BA) | 5 Sec | - | - | - | - |
| AA-1 | B (BA) | 15 Sec | - | - | - | - |
| AA-1 | B (BA) | 30 Sec | - | - | - | - |
| AA-1 | B (BA) | 60 Sec | - | - | - | - |
| AA-1 | B (BA) | 2 Min | **W** | - | - | - |
| AA-1 | B (BA) | 15 min | **+** | - | - | **+** |
| AA-1 | B (BA) | 45 Min | **+** | **+** | **+** | **+** |

Tables 16A-16B depict the results of exposure of Fusobacterium Nucleatum ATCC (FU-3) to various light sources for periods of time between 5 seconds and 45 minutes (900 seconds). FU-3 is a key component of periodontal plaque due to its abundance and its ability to coaggregate with other species in the oral cavity. In Table 16A, results of exposure to no light, and fiber optic sources of white light, FI Pro Light-2mm, and AWP Pro Light-2mm are depicted. In Table 116B, results of exposure to tip mounted light sources at dominant wavelengths of 400 nm (two samples), 590 nm, and a surface mount white light are presented.

| Table 16A - FU-3 with Fiber Optic Source | | | | | | |
|---|---|---|---|---|---|---|
| Organism | Plate | Time | White light-3mm | No light | FI Pro Light-2mm | AWP Pro Light-2mm |
| FU-3 | A (BA) | 5 Sec | - | - | - | - |
| FU-3 | A (BA) | 15 Sec | - | - | - | - |
| FU-3 | A (BA) | 30 Sec | - | - | - | - |
| FU-3 | A (BA) | 60 Sec | - | - | - | - |
| FU-3 | A (BA) | 2 Min | **+** | - | - | - |
| FU-3 | A (BA) | 15 min | **+** | **-** | **+** | no data |
| FU-3 | A (BA) | 45 Min | **+** | **-** | **+** | no data |

| Table 16B - FU-3 With Tip Mounted Source | | | | | | |
|---|---|---|---|---|---|---|
| Organism | Plate | Time | 400 nm | 400 nm | 590 A | Surface mount |
| FU-3 | B (BA) | 5 Sec | - | - | - | - |
| FU-3 | B (BA) | 15 Sec | - | - | - | - |
| FU-3 | B (BA) | 30 Sec | - | - | - | - |
| FU-3 | B (BA) | 60 Sec | - | - | - | - |
| FU-3 | B (BA) | 2 Min | **+** | - | - | - |
| FU-3 | B (BA) | 15 min | **+** | - | - | **W** |
| FU-3 | B (BA) | 45 Min | **+** | - | **W** | **+** |

Table 17 depicts a graph summarizing the efficacy comparison of surface mount radiant energy sources to radiant energy provided by fiber optic delivery on the various organisms presented above in Tables 9A-16B.

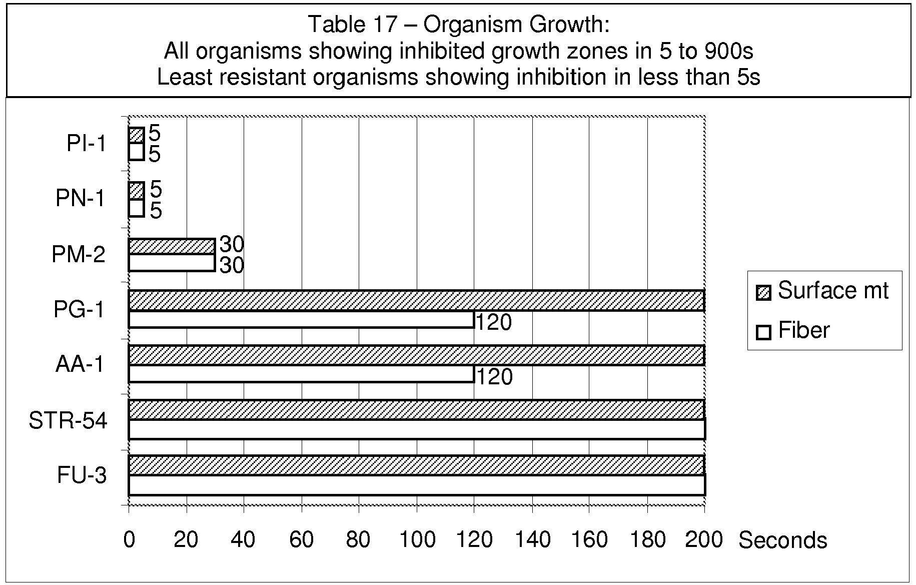

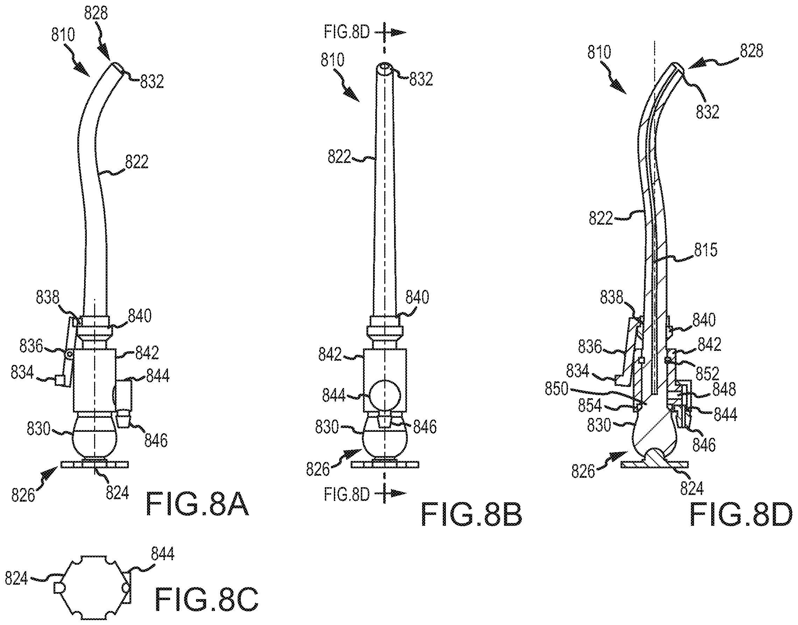

In yet another implementation depicted in Figs. 8A-8D, an integral jet tip 810 forms the water conduit 815 within a molded light tube 822. This configuration allows the jet tip 810 to be smaller closer in size to a standard, non-light emitting tip used on a standard oral irrigator appliance. The one piece all molded design can be produced more economically than multipart designs using a molded water conduit jet tip with an optical fiber or other light tube attached. Further, the coaxial construction allows the tip to be rotated relative to the handle and feature what is not practical in non-coaxial designs.

As shown in Figs. 8A-8D, the jet tip 810 is composed in part of an LED module 824 at a proximal end 826 of the jet tip 810 that shines light into a molded acrylic fiber light injector 830, which in turn focuses this light into the entrance of the molded light tube 822 of the jet tip 810. The light injector 830 is fixed within an opening in a proximal end of a manifold 842 while the light tube 822 is removably inserted within a distal end 828 of the manifold 842. The light injector 830 and the light tube 822 are separated within the manifold 842 by a gap that forms a disk shaped plenum 850 in fluid communication with both the water conduit 815 and a water channel 848 in a water inlet 844 formed as an integral part of or mounted on a sidewall of the manifold 842. The water inlet 844 may form a nipple 846 for attachment of a water line to introduce water from an oral irrigator reservoir into the manifold 842. A distal seal 852, e.g., an O-ring, is located within the manifold 842 to seal against the outer surface of the light tube 822 and prevent water leakage. Similarly, a proximal seal 854, e.g., another O-ring, is located within the manifold 842 to seal against the outer surface of the light injector 830 and prevent water leakage.

The light tube 822 may be further retained within the manifold 842 by a clasp 834 or other retention mechanism. As shown in Figs. 8A, 8B, and 8C, a spring-tensioned clasp 834 may toggle about a hinge 836 mounted on the manifold 842. The clasp 834 may be formed as a claw 838 on the distal end of the clasp 834 to interface with a retention surface 840 formed on the outer wall of the light tube 822. The retention surface 840 may be formed as an annular bulge or shelf surrounding the outer wall of the light tube 822 in order to allow the jet tip 810 to be oriented in any direction when inserted into the manifold 842. While not shown in Figs. 8A-8D, the retention surface 840 may be located along the light tube 822 such that it also interfaces with the distal end of the manifold 842 to indicate that the light tube 822 is fully inserted within the manifold 842 and thereby prevent over-insertion that would prevent formation of the plenum 850.

At the proximal end 826 of the light tube 822, radiant energy is transmitted from the light injector 830 to the light tube 822 and water is also introduced from the plenum 850 into the water conduit 815 formed in the light tube 822. When the plenum 850 is filled with water, the light injector 830 also transmits light into the water as it travels through the water conduit 815. The water in the water conduit 815 thus also provides an additional light conducting structure as well as the cleaning jet of water when emitted from the distal end 828 of the light tube 822. This cylindrical discharged jet stream is substantially laminar and further acts as light tube for the radiant energy. The edges of the laminar stream are bordered by air, which aids in the internal reflection of the light within the water stream, thereby providing tightly focused beam of UV light to the tooth surface. Additionally, the distal end 828 of the light tube 822 may be beveled, faceted, curved, or otherwise configured to focus the radiant energy exiting the light tube 822 to enter the water stream to further enhance the focused beam of light. The water jet further acts to lift the gum tissue away from the tooth surface allowing germicidal light to access the UV photosensitive black-pigmented anaerobic bacteria beneath the gum line.

In an alternate example, a system of lenses may be used to focus light into the end of the light tube 822 rather the molded light injector 830. In other examples, the molded light injector 830 could be replaced by a straight glass or plastic rod with a polished end placed in close proximity the light emitting die of the LED 824. While functional, a disadvantage of this design is that the LED 824 must be obtained in a non standard configuration in order to allow the end of such a glass or plastic rod to be placed in the required close proximity. Further, there is a decrease in efficiency as the analysis below suggests.

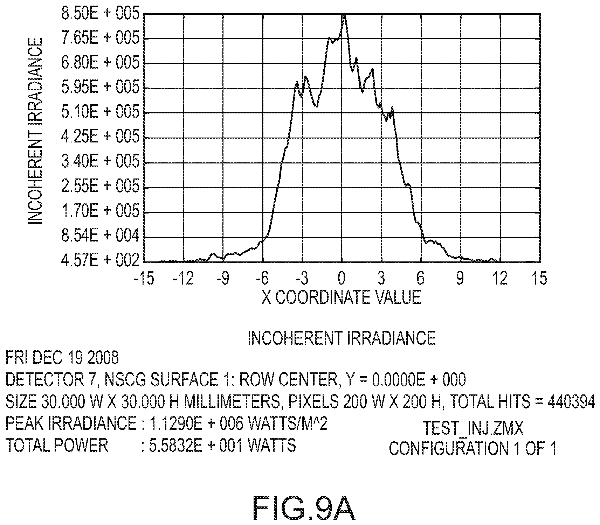

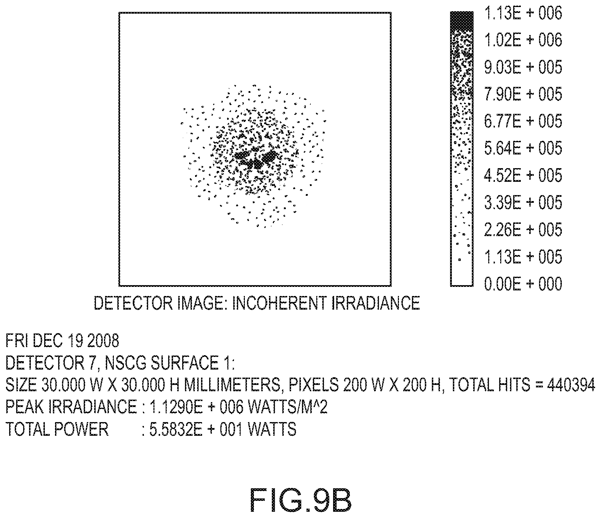

The effectiveness of the oral irrigator device with integral radiant energy delivery system of Figs. 8A-8D is shown in the computer simulation report of Figs. 9A-11B. These reports also demonstrate the focusing ability of the light carrying water stream. In the first configuration presented in Figs. 9A and 9B, A 1 x1 mm, 405nm LED was used as the light source. The jet tip 810 was tapered and curved with 1 mm water gap in the plenum 850. Water was in the water conduit 815 of the jet tip 810, but was not flowing to extend to the tooth surface. The target/detector size was 30x30 mm and was placed 5 mm from distal end 828 of the jet-tip 810. A mask with a hole was placed near the end of the jet-tip 810, to eliminate scattered energy. Fresnel and absorption losses are considered. The LED power is "set" to 100 watts. The incoherent irradiance plot shown in Fig. 9A is in Watts/m². In this experiment, 55.8 watts reaches the detector. The peak irradiance measured at the center of the target was 8.5x10⁵ Watts/m². The highest irradiance calculated for a single location was 1.1290X10⁶ Watts/m². The energy spot as shown in Fig. 9B is approximately 11.8 mm diameter, where >10% of the total energy output was imparted to the peak location.

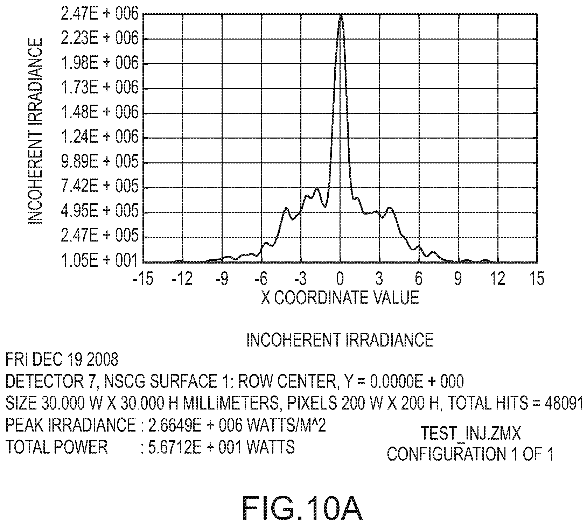

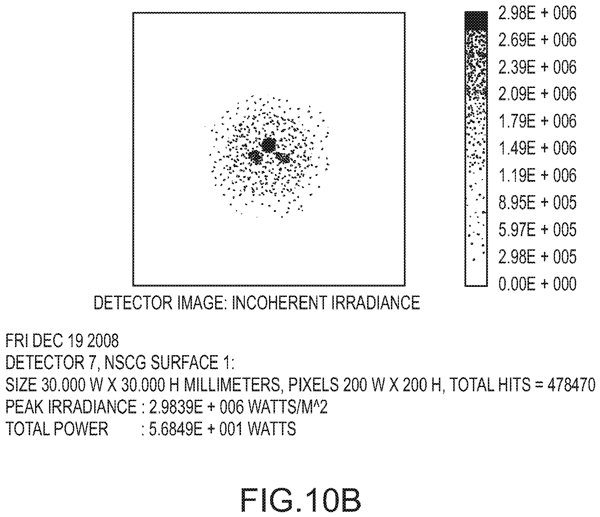

The results of a second configuration are presented in Figs. 10A and 10B. The light source 724 and the jet tip configuration are the same as the configuration corresponding to Figs. 9A and 9B, but in this experiment, the water stream was flowing and extended to target/detector as it would be in actual use. In this experiment, 56.8 watts reached the detector. The peak irradiance measured at the center of the target was 2.5x10⁶ Watts/m², which is three (3) times that of the configuration represented in Figs. 9A and 9B. The energy spot as shown in Fig. 10B is more focused at approximately 9.8 mm diameter, where >10% of the total energy output was imparted to the peak location. This experiment is demonstrative of the enhancement of the bactericidal effect if the water stream is also used to focus the radiant energy on the oral tissue.

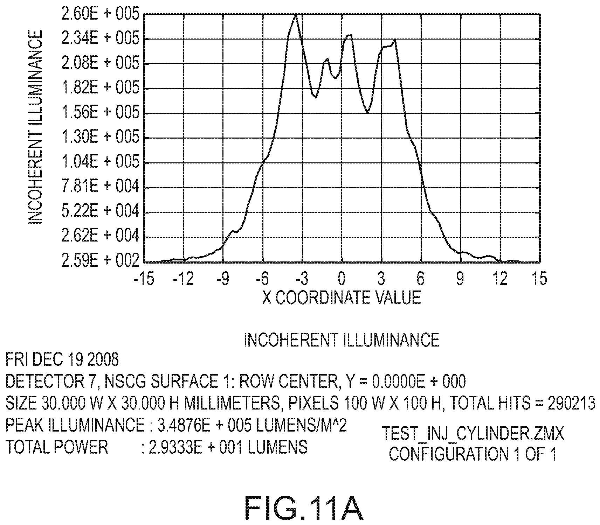

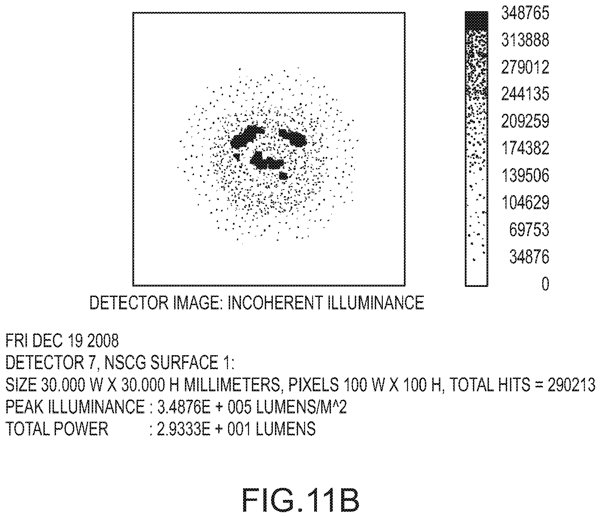

The results of a third configuration are presented in Figs. 11A and 11B. The light source 724 and the jet tip configuration are the same as the configuration corresponding to Figs. 9A and 9B, except that the light injector optic was replaced by a simple cylinder formed of PMMA. Also, as in the first configuration, water was in the water conduit 815 of the jet tip 810, but was not flowing to extend to the tooth surface. In this experiment, 29 watts reached the detector. Also in this experiment, the energy at the detector was measured in illuminance rather than irradiance to provide an alternate method of quantizing the effectiveness. The peak illuminance measured at the center of the target was 2.6x10⁵ lm/m² of energy. The highest illuminance calculated for a single location was 3.48X105 lm/m². The energy spot as shown in Fig. 11B is less focused at approximately 17 mm diameter, where >10% of the total energy output was imparted to the peak location.

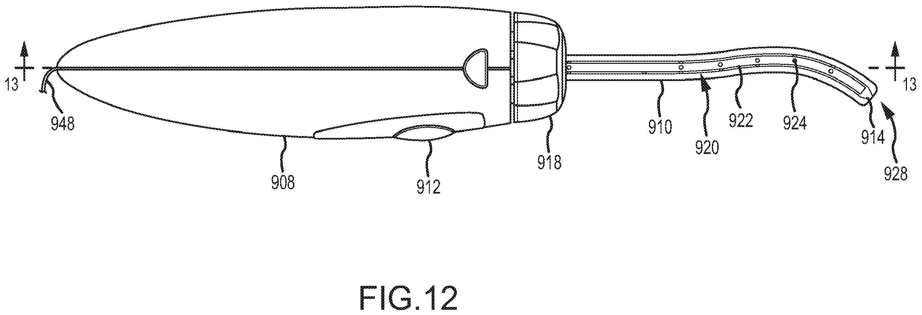

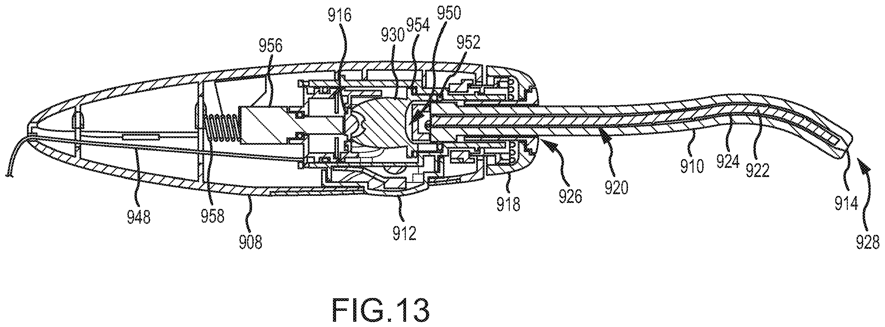

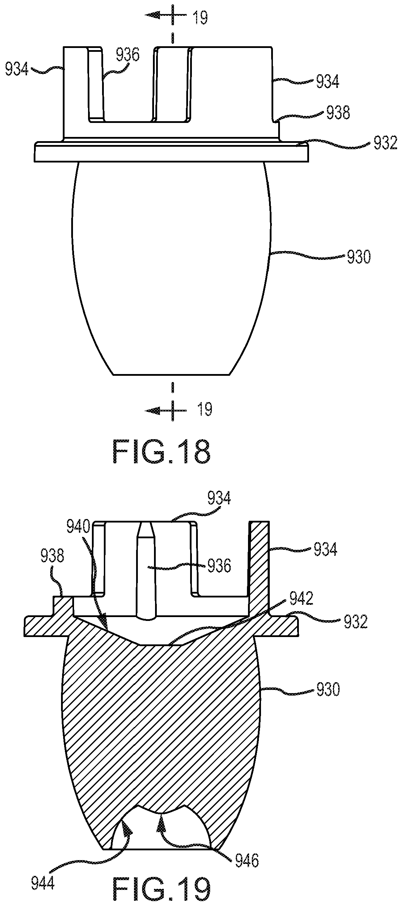

Figs. 12-19 depict an embodiment of the jet handle 908 for use with an oral irrigator system to provide a combination of a fluid stream and radiant energy to an oral cavity. As shown in Figs. 12 and 13, a jet tip 910 extends from the distal end of the jet handle 908 and a fluid conduit 948 connects the jet handle 908 to a pump and fluid reservoir in the base unit (not shown). In addition, a control wire may also extend between the jet handle 908 and the base unit to allow the user to control the pump, the radiant energy source, or both, via one or more actuators 912 located on the jet handle 908. A retention cap 918 holds the jet tip 910 together with the jet handle 908 and allows for removal and replacement of the jet tip 910 as necessary.

The jet tip 910 is provided as a hollow conduit with a proximal end 926 that is received within the jet handle 908 and a distal end 928 that tapers slightly in diameter as compared to the proximal end 926. A light guide 922 extends coaxially within the lumen of the jet tip 910. The light guide receives the radiant energy from a light source (as further described below) and, as a result of an index of refraction of the material forming the light guide 922, the light energy is internally reflected within the light guide 922 such that it does not escape until it reaches the distal end 928. The light guide 922 is of a smaller outer diameter than the diameter of the lumen of the jet tip 910 and similarly tapers in diameter. The space between the outer surface of the light guide 922 and the inner diameter of the jet tip 910 forms a fluid channel 920. In operation, the fluid pumped by the oral irrigator exits the jet tip 910 through an outlet 914 on the distal end 914. At this location, the light energy exits the light guide 922 and is carried within the fluid stream exiting the jet tip 910. The fluid stream is laminar in form and similarly internally reflects the light exiting the light guide 922 to deliver the radiant energy to the same location as the fluid stream.

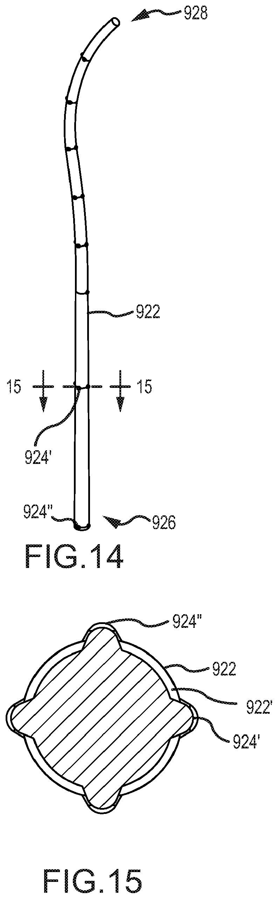

Figs. 14 and 15 show the light guide 922 independently and in greater detail. A plurality of bumps 924 is formed on an outer surface of the light guide 922. The bumps 924 are provided frictionally fit the light guide 922 within the jet tip 910 and to maintain uniform spacing between the outer surface of the light guide 922 and the inner wall of the jet tip 910 to provide the fluid channel 920 within the jet tip 910. There is no set number of or location for the bumps 924 required. As shown in Fig. 14, the bumps may be spaced at various distances longitudinally as well as locations circumferentially. Also, as shown in Fig. 15, the outer surface 922' of the light guide 922 is larger at the proximal end and tapers toward the distal end. This is evident in the differing radii of the bumps 924" at the base of the light guide 922 as compared to the bumps924' further distally along the light guide 922. In the embodiment shown, locations for the bumps 924 were selected to ensure the water channel 920 remains open along the entire length of the jet tip 910. It is desirable to minimize the number of bumps 924 on the light guide to minimize the obstacles within the fluid channel 920 and to optimize the internal reflection of the light within the light guide 922.

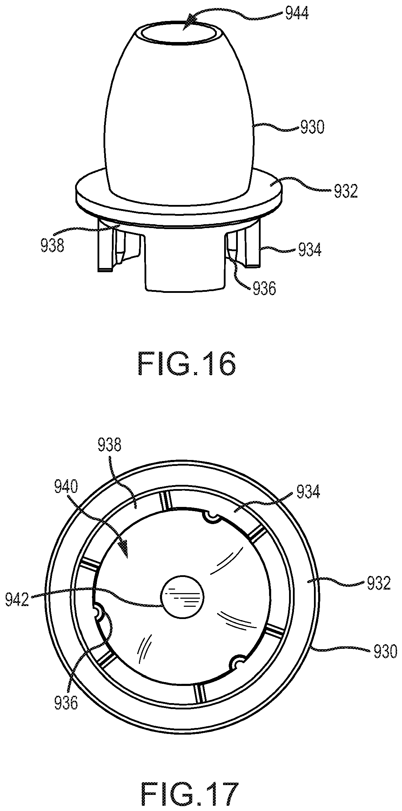

A light source 916, e.g., an LED emitting light at a desired wavelength or over a desired bandwidth, is mounted within the jet handle 908 below the proximal end of the jet tip 910. A heat sink 956, e.g., an aluminum block, may be held in compression with the light source 916 by a spring bias 958 in order to cool the LED light source 916 when in operation. A collimator 930 is mounted between the light source 916 and the proximal end of the light guide 922. The collimator 930 is shown in greater detail in Figs. 16-19. The proximal end of the collimator 930 functions as a collector having a concave surface 944 that transitions into a convex surface 946 to collect and focus the light from the light source 916. In exemplary embodiments, the radius of the sidewalls of the collimator 930 may be between 0.5-1.5 degrees. In the embodiment of Figs. 16-19, the radius is approximately 0.68 degrees. The distal end of the collimator is formed as a lens with a flat base 942 and a distally extending conical sidewall 940 that may be between 20°-30° for best effect. In the embodiment of Figs. 16-19, the angle of the conical sidewall 940 with respect to the base 942 is approximately 23.7 degrees.

A superstructure extends above the distal end of the collimator 930 forming a circumferential flange 932 and a plurality of tabs 934. In the embodiment shown, three tabs 934 are spaced equidistantly around the output lens of the collimator 930 to define a plenum 950 for receipt of fluid from the fluid conduit 948 and injection of the fluid into the water channel 920. A vertical boss 936 is formed on an inner wall of each of the tabs 934 for interfacing with the proximal end of the jet tip 910. A proximal seal 952, e.g. an O-ring, is positioned upon the distal side of the flange 932 to seal the plenum 950 area with respect to an internal housing structure. A lip 938 may extend between each of the tabs 934 adjacent the flange 932 to aid in maintaining the position of the proximal seal 952 when placed under pressure. The spring bias 958 also provides a sealing pressure on the collimator 930 to assist in sealing the plenum 950. A distal seal 954, e.g., and O-ring, is positioned on the distal ends of the tabs 934 to engage with an internal housing structure and an outer wall of the jet tip 910 to provide a sidewall seal for the distal end of the plenum 950.

In operation, the jet handle of the embodiment of Figs. 12-19 flows fluid through the fluid conduit 948 into the plenum 950, and within the water channel 920 in the jet tip 910. When the light source 916 is activated, the light energy is collected by the collimator 930 for a focused output through the plenum and into the proximal end of the light guide 922. The light travels through the light guide 922 and exits the distal end where it is within the water stream exiting the outlet 914 of the jet tip 910. A combination of a pressurized water stream and effective radiant energy is thus delivered simultaneously and coaxially at a common location within the oral cavity.

All directional references (e.g., proximal, distal, upper, lower, upward, downward, left, right, lateral, front, back, top, bottom, above, below, vertical, horizontal, clockwise, and counterclockwise) are only used for identification purposes to aid the reader's understanding of the present invention, and do not create limitations, particularly as to the position, orientation, or use of the invention. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. The exemplary drawings are for purposes of illustration only and the dimensions, positions, order and relative sizes reflected in the drawings attached hereto may vary.

The above specification, examples and data provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of this invention. In particular, it should be understood that the described technology may be employed independent of a personal computer. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the invention as defined in the following claims.

## Claims

1. An oral irrigator (100) comprising
a pump mechanism;
a reservoir (104) in fluid communication with the pump mechanism;
a jet tip (910) in fluid communication with the pump mechanism and configured for directing a fluid pumped from the reservoir (104) by the pump mechanism through the jet tip (910) at a surface inside an oral cavity, the jet tip (910) further comprises a radiant energy conduit (322) having a proximal end and a distal end and the jet tip defines a fluid conduit (948) coaxial with and housing the radiant energy conduit (322) whereby fluid flows through the jet tip (910) in a space between the fluid conduit (948) and the radiant energy conduit (322);
a radiant energy source (524) configured to produce radiant energy and
a collimator (930) positioned between the radiant energy source (524) and the radiant energy conduit (322), the collimator (930) configured to collimate the radiant energy from the radiant energy source (524) and direct the collimated radiant energy at the surface inside the oral cavity; wherein
the proximal end of the jet tip (910) is positioned adjacent the collimator source to capture the collimated radiant energy from the collimator (930) and the distal end directs the collimated radiant energy in generally the same direction as the fluid.

2. The oral irrigator (100) of claim 1, wherein the radiant energy source (524) and the jet tip (910) are of unitary construction to direct both the fluid and the collimated radiant energy in generally the same direction.

3. The oral irrigator (100) of claims 1 or 2, wherein the reservoir (104), the pump mechanism, the jet tip (910), the collimator (930), and the radiant energy source (524) are integrated as a generally unitary combination.

4. The oral irrigator (100) of claims 1 or 2 further comprising a base configured to rest on a support surface and defining a storage position;
a handle (108) configured to rest in the storage position; and wherein
the jet tip (910), the collimator (930), and the radiant energy source (524) are included on
the handle (108), which is movable from the storage position on the base to a second position at least partially removed from the base.

5. The oral irrigator of claim 1, wherein the radiant energy conduit (322) further comprises a plurality of bumps (924) on an outer surface of the radiant energy conduit (322) to maintain a substantially constant separation distance between the radiant energy conduit (322) and the fluid conduit (948).

6. The oral irrigator of claim 1, wherein the radiant energy source (524) is a LED and generates radiant energy between 350-450 nm.

## Patentansprüche

1. Munddusche (100), aufweisend:
einen Pumpenmechanismus;
ein Reservoir (104) in strömungstechnischer Verbindung mit dem Pumpenmechanismus;
eine Düsenspitze (910) in strömungstechnischer Verbindung mit dem Pumpenmechanismus und ausgebildet, ein Fluid, das durch den Pumpenmechanismus aus dem Reservoir (104) gepumpt wird, durch die Düsenspitze (910) auf eine Oberfläche im Inneren einer Mundhöhle zu richten, wobei die Düsenspitze (910) ferner eine Strahlungsenergieleitung (322) mit einem nahen Ende und einem fernen Ende aufweist und die Düsenspitze eine Fluidleitung (948) definiert, die koaxial mit der Strahlungsenergieleitung (322) ist und diese aufnimmt, wobei Fluid durch die Düsenspitze (910) in einem Raum zwischen der Fluidleitung (948) und der Strahlungsenergieleitung (322) strömt;
eine Strahlungsenergiequelle (524), die ausgebildet ist, Strahlungsenergie zu erzeugen, und
einen Kollimator (930), der zwischen der Strahlungsenergiequelle (524) und der Strahlungsenergieleitung (322) positioniert ist, wobei der Kollimator (930) ausgebildet ist, die Strahlungsenergie von der Strahlungsenergiequelle (524) zu kollimieren und die kollimierte Strahlungsenergie auf die Oberfläche im Inneren der Mundhöhle zu lenken; wobei
das nahe Ende der Düsenspitze (910) neben der Kollimatorquelle positioniert ist, um die kollimierte Strahlungsenergie vom Kollimator (930) aufzufangen, und das ferne Ende die kollimierte Strahlungsenergie im Allgemeinen in dieselbe Richtung lenkt wie das Fluid.

2. Munddusche (100) nach Anspruch 1, wobei die Strahlungsenergiequelle (524) und die Düsenspitze (910) eine einheitliche Konstruktion sind, um sowohl das Fluid wie auch die kollimierte Strahlungsenergie im Allgemeinen in dieselbe Richtung zu lenken.

3. Munddusche (100) nach Anspruch 1 oder 2, wobei das Reservoir (104), der Pumpenmechanismus, die Düsenspitze (910), der Kollimator (930) und die Strahlungsenergiequelle (524) als eine im Allgemeinen einheitliche Kombination integriert sind.

4. Munddusche (100) nach Anspruch 1 oder 2, ferner aufweisend
eine Basis, die ausgebildet ist, auf einer Trägerfläche zu liegen und eine Lagerungsposition zu definieren;
einen Griff (108), der ausgebildet ist, in der Lagerungsposition zu liegen; und wobei die Düsenspitze (910), der Kollimator (930) und die Strahlungsenergiequelle (524) auf dem Griff (108) enthalten sind, der aus der Lagerungsposition auf der Basis in eine zweite Position beweglich ist, die zumindest teilweise von der Basis entfernt ist.

5. Munddusche (100) nach Anspruch 1, wobei die Strahlungsenergieleitung (322) ferner mehrere Höcker (924) auf einer Außenfläche der Strahlungsenergieleitung (322) aufweist, um einen im Wesentlichen konstanten Trennungsabstand zwischen der Strahlungsenergieleitung (322) und der Fluidleitung (948) aufrechtzuerhalten.

6. Munddusche nach Anspruch 1, wobei die Strahlungsenergiequelle (524) eine LED ist und Strahlungsenergie zwischen 350-450 nm generiert.

## Revendications

1. Appareil irrigateur buccal (100) comprenant un mécanisme de pompe ;
un réservoir (104) en communication fluidique avec le mécanisme de pompe ;
une pointe à jet (910) en communication fluidique avec le mécanisme de pompe et configurée pour orienter un fluide pompé à partir du réservoir (104) par le mécanisme de pompe à travers la pointe à jet (910) sur une surface à l'intérieur d'une cavité buccale, la pointe à jet (910) comprenant en outre un conduit d'énergie radiante (322) ayant une extrémité proximale et une extrémité distale et la pointe à jet définissant un conduit de fluide (948) coaxial avec le conduit d'énergie radiante (322) et recevant ce dernier, du fluide s'écoulant ainsi à travers la pointe à jet (910) dans un espace entre le conduit de fluide (948) et le conduit d'énergie radiante (322) ;
une source d'énergie radiante (524) configurée pour produire de l'énergie radiante et
un collimateur (930) positionné entre la source d'énergie radiante (524) et le conduit d'énergie radiante (322), le collimateur (930) étant configuré pour collimater l'énergie radiante provenant de la source d'énergie radiante (524) et diriger l'énergie radiante collimatée au niveau de la surface intérieure de la cavité buccale ;
l'extrémité proximale de la pointe à jet (910) étant positionnée en position adjacente à la source du collimateur pour capturer l'énergie radiante collimatée provenant du collimateur (930) et l'extrémité distale dirigeant l'énergie radiante collimatée généralement dans la même direction que le fluide.

2. Appareil irrigateur buccal (100) selon la revendication 1, dans lequel la source d'énergie radiante (524) et la pointe à jet (910) sont de construction unitaire afin de diriger à la fois le fluide et l'énergie radiante collimatée généralement dans la même direction.

3. Appareil irrigateur buccal (100) selon les revendications 1 ou 2, dans lequel le réservoir (104), le mécanisme de pompe, la pointe à jet (910), le collimateur (930) et la source d'énergie radiante (524) sont intégrés sous forme d'une combinaison généralement unitaire.

4. Appareil irrigateur buccal (100) selon les revendications 1 ou 2, comprenant en outre une base configurée pour reposer sur une surface de support et définissant une position de stockage ;
une poignée (108) configurée pour reposer dans la position de stockage ; et
la pointe à jet (910), le collimateur (930), et la source d'énergie radiante (524) étant inclus sur la poignée (108), qui peut être déplacée de la position de stockage sur la base dans une deuxième position au moins en partie éloignée de la base.

5. Appareil irrigateur buccal selon la revendication 1, dans lequel le conduit d'énergie radiante (322) comprend en outre une pluralité de bosses (924) sur une surface extérieure du conduit d'énergie radiante (322) pour maintenir une distance de séparation sensiblement constante entre le conduit d'énergie radiante (322) et le conduit de fluide (948).

6. Appareil irrigateur buccal selon la revendication 1, dans lequel la source d'énergie radiante (524) est une DEL et génère de l'énergie radiante entre 350 et 450 nm.
